# EUROPEAN PATENT APPLICATION

(11) **EP 1 726 300 A1**
(43) Date of publication of application: **29.11.2006**
(21) Application number: 05300406.5
(22) Date of filing: 24.05.2005
(51) Int. Cl.: A61K 9/50, A61K 45/06

(54) **Oral pharmaceutical formulation for the prevention and/or the treatment of cardiovascular and inflammatory diseases**

(71) Applicant: FLAMEL TECHNOLOGIES, 69693 Venissieux Cédex (FR)
(72) Inventor: Soula, Gérard, 69330 Meyzieu (FR); Guimberteau, Florence, 33450 Montusson (FR); Meyrueix, Rémi, Le Bois Saint Rambert, Lyon 69009 (FR)
(74) Representative: Fleurance, Raphaël

(57) **Abstract**

The invention relates to oral pharmaceutical formulations for the prevention and/or the treatment of cardiovascular and inflammatory diseases. This invention also addresses methods of prevention and/or treatment of these diseases, using these oral formulations. Cardiovascular diseases of particular concern for the invention are the diseases resulting from excessive and uncontrolled platelet aggregations (platelet disorders). More particularly, these diseases are those caused by an excess of thromboxane and against which antithrombotic treatments can be proposed to the patients.

## Description

The invention relates to oral pharmaceutical formulations for the prevention and/or the treatment of cardiovascular and inflammatory diseases. This invention also addresses methods of prevention and/or treatment of these diseases, using these oral formulations. Cardiovascular diseases of particular concern for the invention are the diseases resulting from excessive and uncontrolled platelet aggregations (platelet disorders). More particularly, these diseases are those caused by an excess of thromboxane and against which antithrombotic treatments can be proposed to the patients.

In the present exposure, the "thrombotic" troubles denotes, notably, the thrombotic cardiovascular events such as such as stroke, myocardial ischemia, myocardial infarction, angina pectoris, transient ischemic attack, reversible ischemic neurological deficits, and any similar thrombotic event in any vascular bed (splanchnic, renal, aortic, peripheral, etc.).

One of the medical problems supporting the instant invention is the enhancement of the safety of these antithrombotic treatments.

Indeed, to inhibit platelet aggregation and limit the inherent cardiovascular risks, it is known to administrate to the patients two major anti-platelets drugs, namely low dose aspirin and PLAVIX®.

PLAVIX® (clopidogrel bisulfate) is an inhibitor of platelet aggregation acting by direct inhibition of adenosine diphosphate (ADP) binding to its receptor and of the subsequent A.P. mediated activation of the glycoprotein GPIIb/ IIIa complex. Chemically, it is methyl (+)-( S)-a -(2- chlorophenyl)-6,7-dihydrothieno[3,2-c] pyridine-5(4H)-acetate sulfate (1:1). The side effects of the PLAVIX® are notably: gastrointestinal hemorrhaging; Neutropenia/agranulocytosis; gastrointestinal events (e. g. abdominal pain, dyspepsia, gastritis and constipation, peptic, gastric or duodenal ulcers, diarrhea); Rash and Other Skin Disorders, etc.

Aspirin, or acetylsalicylic acid, acts to prevent platelet aggregation by irreversibly inhibiting cyclooxygenase (COX-1 & COX-2, collectively known as COX). COX converts arachodonic acid to thromboxane, a potent vasoconstrictor and a platelet aggregation stimulator. Aspirin inhibits COX by acetylating it. The inhibition of COX activity by aspirin is generally irreversible. This is an important distinction for aspirin because the duration of the effects of aspirin is related to the turnover of COX in different tissue targets. Platelets are especially susceptible to aspirin mediated irreversible inactivation of COX because platelets have little or no capacity for protein biosynthesis and, thus, cannot regenerate the COX enzyme. In practical terms, this means that a single dose of aspirin will inhibit the platelet COX for the life of the platelet, 8-11 days.

When aspirin is absorbed from the digestive tract, it is collected by the portal vein. The portal vein then goes to the liver where the aspirin is deacetylated. Once deacetylated, aspirin no longer has the ability to aceytelate COX. However, the mechanism in the liver can rapidly reach saturation causing the aspirin overflow to enter the systemic blood circulation. In the systemic circulation, aspirin that has not been deacetylated by the liver can further inhibit COX in other tissues and cells. For instance, in the endothelial cells that line the vasculature, aspirin-induced COX inhibition results in a decrease of prostacyclin, which is a potent vasodilator, a platelet aggregation inhibitor and a cytoprotector. Therefore, aspirin that enters the systemic blood circulation results in inhibition of the prostacyclin and other prostaglandins, which runs counter to the desired effect on platelet aggregation. This phenomenon of blind inhibition of the different prostaglandins in the organism is commonly referred to as the dilemma of aspirin. It is well known to scientists and has been widely described in the literature.

Aspirin is a very useful medication for the prevention of cardiovascular thrombotic events in patients with or those at risk for cardiovascular disease. However, there are serious side effects of aspirin administration. For example, the most common side effect is a propensity to induce gastric or intestinal ulceration, which may result in hemorrhaging. This effect occurs when acetylated aspirin allows back diffusion of acid into the gastric mucosa and induces tissue damage and bleeding, correlated with inhibition of the biosynthesis of gastric prostaglandins that ordinarily serve as cytoprotective mucous in the intestines. The gastrointestinal effects of aspirin may be caused by its lack of selectivity between antiplatelet COX-1 inhibition and endothelial COX-1 inhibition leading to gastric mucosal effects.

In patients with established cardiovascular disease, aspirin use has been documented to decrease the risk of a primary myocardial infarction, stroke and vascular death. Secondary prevention refers to the use of aspirin to prevent cardiovascular events in patients with established cardiovascular disease such as a myocardial infarction, stroke, or angina. The use of aspirin in these individuals is recommended based on a documented decrease in future cardiovascular events and mortality. The risk for gastrointestinal injury is observed in patients being treated with aspirin, even at dosages as low as 81 mg/day for cardio protection.

In patients with established cardiovascular disease, aspirin may have further side effects. For example, aspirin may decrease renal blood flow and the rate of glomerular filtration in patients with congestive heart failure. Therefore, acute renal failure may be precipitated. Aspirin may also promote the retention of salt and water by reducing the prostaglandin induced inhibition of both the reabsorption of chloride and the action of anti-diuretic hormone. This may cause edema in some patients who are treated with aspirin and may reduce the effectiveness of anti-hypertensive regimens. Aspirin and other COX-2 inhibitors may also increase the risk of heart disease. Metabolism of arachodonic acid by COX-2 results in the production of prostaglandins, which promotes inflammation. Therefore inhibition of COX-2 results in decreased inflammation. However, COX-2 inhibition also promotes arachodonic acid to be converted to pro-inflammatory agents such as leukotriene B4 and thromboxane A2. The resulting increase in these pro-inflammatory agents may lead to increased atherosclerosis and platelet aggregation, and other complications such as stroke and heart attack.

Aspirin that is not metabolized by the liver may induce serious side effects. Since aspirin may be taken by the patient for a substantial portion of his life, it is important to improve the safety profile of the treatment for the tens of millions of patients today who regularly take anti-platelet drugs. Thus, it appears that there is a need in a therapeutic solution for treating the pathologies linked to platelet aggregation, optionally the inflammatory disorders, without causing the serious side effects to the patients.

US-B-6,599,529 relates to an oral pharmaceutical modified release multiple-units composition for the administration of a therapeutically and/or prophylactically effective amount of a non-steroid anti-inflammatory drug substance - e.g., aspirin - (in the following abbreviated "an NSAID substance") to obtain both a relatively fast or quick onset of the therapeutic effect and the maintenance of a therapeutically active plasma concentration for a relatively long period of time. The modified release multiple-units composition comprises at least two fractions of multiple units such as a first and a second fraction. The first fraction is an immediate release form of NSAIDs, which comprises individual units that are designed to quickly release the drug substance. The second fraction is a delayed release sustained release form, which comprises individual units that are designed to slowly release the drug substance to enable a delayed and extended release of the drug substance. Typically, the second fraction comprises multiple units which are coated with a sustained release coating designed to release the drug substance in such a manner that the maintenance of a therapeutically active plasma concentration for a relatively long period of time are obtained (once- or twice-a-day administration)- [Pellet cores: Polysorbate 20/Cellulose microcrystalline/Lactose/Carmellose sodium/Maltodextrin/Pregelatinized starch -Inner coat: Hypromellose(Methocel E prem) /Magnesium stearate / Talc / Eudragit NE 30 D - Outer coat: Hypromellose (Methocel E5 prem) / Talc].The composition can comprise a further active drug substance selected from the group consisting of an antidepressant, an opioid, a prostaglandin analog, a glucocorticosteroid, a cytostaticum, a H₂ receptor antagonist, a proton pump inhibitor and an antacid. The immediate release fraction of NSAIDs (aspirin) in this composition is still creating all gastric side effects mentioned before. The problem of aspirin dilemma is not solved.

US-A-2004/0121004 & US-A-2004/0131676 disclose a non-enterically coated dosage form comprising: a) a proton pump inhibitor(lansoprazole); b) a buffer; and c) a non-steroidal anti-inflammatory drug (aspirin at an amount between 50 mg and 100 mg) and a method of treating a condition selected from the group consisting of angina, aorto-pulmonary shunt occlusion, colorectal cancer, esophageal cancer, colon cancer, coronary artery disease, dementia, dysmenorrhea, myocardial infarction, rheumatoid arthritis, osteoarthritis, pain, headache, migraine headache, stroke, thrombocythemia, post-operative thromboembolism, ischemia, bursitis, cognitive decline, fever, gout, musculoskeletal disorders, soft tissue injury, and pericarditis; wherein the method comprises administering to a patient having one or more of the above conditions said non-enterically coated dosage form. This form is an immediate release form of acetylsalicylic acid at basic pH. Granulates of NSAIDs are prepared from Magnesium Hydroxide- buffer-; Calcium Carbonate; Mannitol; Avicel (micro-crystalline Cellulose); PVPP (Cross- povidone). These granulates are tabletted.

The immediate release aspirin included in this dosage and the immediate release fraction of NSAIDs (aspirin) in this composition still creates many of the gastric side effects mentioned before. The problem of aspirin dilemma is not solved.

Finally, the prior art discloses how treating the side effects in terms of bleeding and pitichia induced by acetylsalicylic acid entering the systemic circulation. This prior art proposes to co-administrate large amounts of proton pump inhibitor to increase the gastric pH to reduce the pain. The drawbacks of this approach are as follows:
1. damages of the gastric mucosa are very high and permanent;
2. the large dose of proton pump inhibitor results in a constant high pH, so which is deleterious for chronic use.

US-B-5,603,957 belonging to the applicant, and incorporated in its entirety by reference discloses and claims a pharmaceutical form comprising microcapsules for the controlled release of acetylsalicylic acid in the gastrointestinal environment, said microcapsules consisting of particles of acetylsalicylic acid with a size of between 100 and 1000 µm. These microcapsules are coated and designed so that, when ingested orally in a single administration of a dose of between 50 and 325 mg of acetylsalicylic acid, they induce moderate acetylsalicylic acid absorption kinetics in vivo in man, extending over at least 24 hours, said acetylsalicylic acid absorption being less than or equal to 10% by weight of the absorbed fraction of the dose at a time t after ingestion of 0.4 hour, less than or equal to 50% by weight of the absorbed fraction of the dose at t=3.9 hours, and less than or equal to 90% by weight of the absorbed fraction of the dose at t=23 hours, t being given to within +/-10%. It is also possible to create microcapsules of even smaller size, such as 50µm or less. One method to create such smaller microcapsules is disclosed in USPN 6,022,562 to Durant et al., which is incorporated by reference in its entirety.

We find it surprising that we can use the pharmaceutical form of microcapsules to selectively inhibit COX in platelets, and thus reduce the production of thromboxane, while minimizing COX inhibition in the circulation. It is therefore possible to optimize the inhibition of platelet aggregation, in order to prevent and/or treat cardiovascular diseases and risks, while minimizing the side effects.

While not wishing to be constrained by any mode of action, we believe that by coating the acetylsalicylic acid to form microcapsules, we promote the direct action of aspirin on only the COX of the blood platelets in the portal circulation. Our pharmaceutical formulation results in a low, constant release rate of aspirin being released from the microcapsules and absorbed by the portal vein. This low release rate of aspirin is sufficient to inhibit COX in the portal vein, and therefore prevent thromboxane formation and platelet aggregation. Once aspirin is deacetylated, it is no longer active in that it can no longer inhibit COX. The liver can quickly be saturated with aspirin, and any aspirin that is not deacetylated will overflow into the systemic blood stream. We find it surprising that our use of microcapsules results in a release rate of aspirin low enough such that the liver is not saturated. Therefore, once the low release rate of aspirin inhibits the COX in the platelets of the portal vein, any remaining active aspirin is then deacetylated by the liver. This results in minimal aspirin overflow into the systemic circulation to inhibit prostaglandin and prostacyclin production, thus preventing damage of the gastroendothelium and other side effects of aspirin. Thus, the method permits the rapid saturation of the liver without the normal deleterious side effects, a result that we find unexpected.

We also propose combining a controlled release form of aspirin that releases a low, constant release rate of aspirin such that very little aspirin reaches the systemic circulation, and a minimal release rate of a gastric acid suppressing agent to decrease the gastric damages by increasing the gastric pH.

While not wishing to be constrained to low dose of gastric acid suppressing agent, we fmd it surprising that we can greatly minimize gastric damage with only a minimal dose of gastric acid suppressing agent. The low dose of gastric acid suppressing agent in our invention increases the gastric pH only a small amount. Further, the low, constant dose of aspirin released in the intestinal tract would sufficiently inhibit COX in the platelets while having minimal effects on the systemic prostaglandins. Therefore, the applicant takes credit for demonstrating that the combination of a controlled release aspirin microcapsules with at least one gastric acid suppressing agent makes it possible to increase the safety of the anti-platelet drug while decreasing the side effects.

The present invention concerns an oral pharmaceutical formulation for enhancing notably the safety of antithrombotic treatments,
■ comprising a combination of microcapsules for the controlled release of acetylsalicylic acid (acetylsalicylic acid), in the gastrointestinal environment and at least one gastric acid suppressing agent,
■ said microcapsules:
   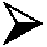 having an in vitro release profile, in 0.05M potassium dihydrogenophosphate/sodium hydroxide buffer medium pH 6.8, such that 70% of the acetylsalicylic acid is released over a period of time of between 2 and 20 hours, preferably between 4 and 18 hours, and even more preferably between 6 and 15 hours, and
   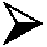 inhibiting COX-1 in portal vein which limits the production of thromboxane while maintaining COX-1 in systemic blood stream and hence limiting the inhibition of the production of prostaglandin and prostacyclin production to decrease the side effects and to maintain its vasodilatation properties;
■ said gastric acid suppressing agent increasing the pH of the stomach just enough to decrease the dissolving of aspirin while minimizing the damage from any residual of non-deacetylated acetylsalicylic acid coming from the liver and the portal blood circulation and entering the systemic blood circulation.

The term "controlled release" denotes, in the present disclosure, a prolonged or sustained release and/or a delayed release and/or a pulsed release of active principle by an oral pharmaceutical formulation. Such a controlled-release oral pharmaceutical formulation may, for example, comprise an immediate-release phase and a slow-release phase. Modified-release medicinal products are well known in this field; see, for example, Remington: The Science and practice of pharmacy", 19th edition, Mack Publishing Co. Pennsylvania, USA. The modified release may in particular be a prolonged and/or delayed release.

Preferably, the microcapsules can be orally ingestible in a dose and can comprise particles of acetylsalicylic acid with a size of less than about 1000 µm, preferably between about 50 µm or 100 µm to 1000 µm, which are coated and designed so that, when ingested orally in a single administration of a dose of between 50 and 325 mg of acetylsalicylic acid, they induce moderate acetylsalicylic acid absorption kinetics in vivo in man, extending over at least 24 hours, said acetylsalicylic acid absorption being less than or equal to 10% by weight of the absorbed fraction of the dose at a time t after ingestion of 0.4 hour, less than or equal to 50% by weight of the absorbed fraction of the dose at t=3.9 hours, and less than or equal to 90% by weight of the absorbed fraction of the dose at t=23 hours, t being given to within +/-10%.

According to another of its features, the present invention also concerns a method for treating the thrombotic diseases, said method comprising the administration to a patient an oral pharmaceutical formulation according to the above definition. Said method is a method of preventing and/or treating pathological disorders associated with excesses of thromboxane, particularly cardiovascular diseases and risks. This method consists in the oral administration of the pharmaceutical formulation according to the invention, preferably in a once or twice-a-day administration.

It is apparent from the foregoing text that the microcapsules of the invention are very effective in pharmacological terms, perfectly tolerated by the organism, especially as regards gastric tolerance, capable of being presented in various appropriate galenical forms and, finally, easy and inexpensive to obtain.

The controlled release-acetylsalicylic acid microcapsules have high selectivity for the thromboxane inhibition, which makes it possible to maintain the production of prostacyclin, in order to protect the gastro-intestinal tract.

Furthermore, the gastric acid suppressing agent maintains the pH of the stomach high enough to reduce the acidic erosion of the surface of the stomach and even to facilitate the healing process when some ulceration occurs.

In a preferred embodiment of the invention, the absorption takes place over a period of between 24 and hours in the following manner:
- 10% of the absorbed fraction of the dose at t=0.4 to 5 hours,
- 50% of the absorbed fraction of the dose at t=3.9 to 25 hours,
- and 90% of the absorbed fraction of the dose at t=23 to 45 hours.

The curve of FIG. 1 of US-B-5,603,957 shows the kinetic profile of the in vivo absorption of acetylsalicylic acid, and more precisely the upper limit of the acetylsalicylic acid in vivo absorption profile induced by the controlled release-acetylsalicylic acid microcapsules according to US-B-5,603,957, as a function of time, at a dose of 320 mg. This absorption is expressed in % absorbed relative to the absorbed fraction of the initial dose D. This curve is obtained by conventional deconvolution analysis (Milo GIBALDI and D. PERRIER, Pharmacokinetics, 2nd ed., New York, Marcel Dekker Inc., 1983, p. 145-167) from the mean curves of the plasma concentrations as a function of time after the oral administration of 350 mg of acetylsalicylic acid equivalents of ASPEGIC. (control form) and 320 mg of acetylsalicylic acid equivalents of microcapsules according to the invention in the form of gelatin capsules. In this case, the tracer molecule chosen for the plasma concentrations as a function of time is necessarily salicylic acid (SA), a metabolite of acetylsalicylic acid. The plasma concentrations of SA are determined by HPLC. The critical points at 0.4, 3.9 and 23 h, given above in the definition of the microcapsules of the invention, are of course to be found on this curve. Beyond this curve, the hepatic acetylsalicylic acid de-acetylation mechanism is saturated. It must be considered that all the acetylsalicylic acid in vivo absorption profiles contained in the area under the curve are controlled release-acetylsalicylic acid microcapsules according to US-B-5,603,957.

To solve the technical problem on which the invention is based, it is highly preferable that the formulation according to the invention be free or almost free of immediate release acetylsalicylic acid form. The term "almost free" means that the formulation can include negligible amount of immediate release acetylsalicylic acid form, namely a insufficient amount so that there is remaining non-deacetylated acetylsalicylic acid in the systemic blood stream after the liver, said non-deacetylated acetylsalicylic acid being in a sufficient amount to inhibit COX-1 of the systemic blood compartment.

"Immediate release acetylsalicylic acid form" is intended to denote, in the present disclosure, a form in which the acetylsalicylic acid is released, at pH 6.8 and under sink conditions in a in vitro dissolution test, most of the amount of acetylsalicylic acid contained in the immediate release acetylsalicylic acid form, in a relatively brief period of time; for example at least 70% of the acetylsalicylic acid is preferably released in forty five minutes and more preferably in thirty minutes.

All the dissolution profiles to which reference is made in the present disclosure are determined according to the indications of the European Pharmacopoeia, 4th edition, entitled: "Dissolution test for solid oral forms": type II dissolutest performed under SINK conditions, at 37°C, at a test dose of 10 mg of active, and with agitation of 100 rpm.

In a preferred embodiment of the invention, the oral pharmaceutical formulation according to the invention, contains a dose of acetylsalicylic acid in the controlled release-acetylsalicylic acid microcapsules, said dose being comprised between 60 and 320 mg. In a more preferred embodiment of the invention, the oral pharmaceutical formulation according to the invention, contains a dose of acetylsalicylic acid in the controlled release-acetylsalicylic acid microcapsules, said dose being comprised between 75 and 310 mg. In another preferred modality of the invention, the oral pharmaceutical formulation according to the invention, contains a dose of gastric acid suppressing agent comprised between 1 and 130 mg.

In a more preferred modality of the invention, the oral pharmaceutical formulation according to the invention, contains a dose of gastric acid suppressing agent comprised between 2 and 120 mg.

According to a first embodiment, the oral pharmaceutical formulation according to the invention has the following characteristics, among others:
a) the dose of acetylsalicylic acid in the controlled release-acetylsalicylic acid microcapsules is comprised between 50 and 325 mg;
b) the dose of gastric acid suppressing agent - preferably a proton pump inhibitor (proton pump inhibitor)- is comprised between 5 and 120 mg;
c) it is a once-a-day administration form.

The oral pharmaceutical formulation comprising a), b), c) features, can include at least one immediate release acetylsalicylic acid form.

The oral pharmaceutical formulation comprising a), b), c) features, can include at least one immediate release gastric acid suppressing agent.

The oral pharmaceutical formulation comprising a), b), c) features, can include at least one controlled release gastric acid suppressing agent.

According to a second embodiment, the oral pharmaceutical formulation according the invention has the following characteristics, among others:
i. wherein the dose of acetylsalicylic acid in the microcapsules is comprised between 50 and 325 mg;
ii. wherein the dose of gastric acid suppressing agent - preferably a proton pump inhibitor (proton pump inhibitor)- is comprised between 5 and 120 mg;
iii. which is a twice-a-day administration form.

The oral pharmaceutical formulation comprising i, ii, iii features, can include at least one immediate release acetylsalicylic acid form.

The oral pharmaceutical formulation comprising i, ii, iii features, can include at least one immediate release gastric acid suppressing agent. The oral pharmaceutical formulation comprising i, ii, iii features, can include at least one controlled release gastric acid suppressing agent.

It is remarkable that the oral pharmaceutical formulation according to the invention, is designed so that it induces reduction of bleeding and pitichia, notably, in the stomach during the treatment.

We find it surprising that we can selectively inhibit COX and platelets in the portal vein permitting minimal aspirin to enter the systemic circulation. This significantly increases the comfort of the patients and the safety of the drug. Patients will be no longer compelled either to interrupt the treatment with aspirin or to switch to another drug. This also permits a method of quickly inhibiting COX in platelets with less side effects than previously known. Finally, we have a method that permits the unexpected precise titration of the effects of aspirin on the liver, the circulatory system, and the stomach.

This remarkable feature can give rise to a method for reducing the side effects during the treatment of diseases at least partially caused by an inhibition of Cox-1 in systemic circulation, said treatment comprising the administration to a patient an oral pharmaceutical formulation including acetylsalicylic acid, wherein the oral pharmaceutical formulation is a formulation for enhancing, notably, the safety of antithrombotic treatments, said formulation comprising a combination of at least one gastric acid suppressing agent - preferably a proton pump inhibitor (proton pump inhibitor)- and microcapsules for the controlled release of acetylsalicylic acid (acetylsalicylic acid) in the gastrointestinal environment,
■ wherein said microcapsules:
   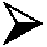 having an in vitro release profile, in 0.05M potassium dihydrogenophosphate/sodium hydroxide buffer medium pH 6.8, such that 70% of the acetylsalicylic acid is released over a period of time of between 2 and 20 hours, preferably between 4 and 18 hours, and even more preferably between 6 and 15 hours, and
   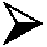 inhibiting COX-1 in portal vein which limits the production of thromboxane while maintaining COX-1 in systemic blood stream and hence limiting the inhibition of the production of prostaglandin and prostacyclin, to protect the gastric endothelium and to maintain its vasodilatation properties;
■ said gastric acid suppressing agent increasing the pH of the stomach just enough to minimize the damage resulting from the residual amount of non-deacetylated acetylsalicylic acid coming from the liver and the portal blood circulation and entering the systemic blood circulation.

The side effects of the oral pharmaceutical formulation according to the invention is also very interesting in that it is designed so that it improves the healing process, notably, in the stomach during the treatment. The oral pharmaceutical formulation is also designed to decrease other side effects of antithrombotic treatments, such as gastric or intestinal ulceration and hemorrhaging, renal failure, edema, atherosclerosis, and any resulting cardiovascular disease.

This interesting feature can give rise to a method according to another aspect of the present invention, namely a method for improving the healing process, notably, in the stomach, during the treatment of diseases at least partially caused by an inhibition of Cox-1 in systemic circulation, said treatment comprising the administration to a patient an oral pharmaceutical formulation including acetylsalicylic acid, wherein the oral pharmaceutical formulation is a formulation for enhancing, notably, the safety of antithrombotic treatments, said formulation comprising a combination of at least one gastric acid suppressing agent - preferably a proton pump inhibitor (proton pump inhibitor)- and microcapsules for the controlled release of acetylsalicylic acid (acetylsalicylic acid) in the gastrointestinal environment,
■ wherein said microcapsules:
   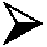 having an in vitro release profile, in 0.05M potassium dihydrogenophosphate/sodium hydroxide buffer medium pH 6.8, such that 70% of the acetylsalicylic acid is released over a period of time of between 2 and 20 hours, preferably between 4 and 18 hours, and even more preferably between 6 and 15 hours, and
   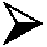 inhibiting COX-1 in portal vein which limits the production of thromboxane while maintaining COX-1 in systemic blood stream and hence limiting the inhibition of the production of prostaglandin and prostacyclin, to protect the gastric endothelium and to maintain its vasodilatation properties;
■ said gastric acid suppressing agent increasing the pH of the stomach just enough to minimize the damage resulting from the residual amount of non-deacetylated acetylsalicylic acid coming from the liver and the portal blood circulation and entering the systemic blood circulation.

In the two above mentioned methods, the implemented microcapsules being orally ingestible in a dose and comprising particles of acetylsalicylic acid with a size of less than 1000 µm which are coated and designed so that, when ingested orally in a single administration of a dose of acetylsalicylic acid, it induces acetylsalicylic acid absorption kinetics in vivo in man, extending over at least 24 hours, said acetylsalicylic acid absorption being less than or equal to 10% by weight of the absorbed fraction of the dose at about 0.4 hour post ingestion, less than or equal to 50% by weight of the absorbed fraction of the dose at about 3.9 hours post ingestion, and less than or equal to 90% by weight of the absorbed fraction of the dose at about 23 hours post ingestion.

Unless otherwise indicated, use of the term "about" in this invention description is intended to mean plus or minus 10% of the designated amount; thus, "about 5 to 80%" would mean a range of 4.5-5.5% to 76-84%.

The gastric acid suppressing agent is preferably a proton pump inhibitor (proton pump inhibitor) or a proton pump inhibitor is a substituted benzimidazole which inhibits gastric acid secretions by specific inhibition of the H⁺, K⁺-ATPase enzymatic system (proton pump) of the secretory surface of parietal gastric cells. A proton pump inhibitor is an advantageous substitute for histamine H2 receptor antagonists (blocking of gastric acid secretion) or for antacids, which are not fully effective in the treatment of ulcers, associated or not with *Helicobacter pylori* infection or of other gastric disorders, and which in addition lead to many side effects.

A proton pump inhibitor is a lipophilic weak base that is poorly soluble in water. It undergoes rapid degradation under acidic conditions but, on the other hand, it is relatively stable at neutral or basic pH.

The proton pump inhibitor more particularly concerned by the present invention are derivates of benzimidazole. These latter are substituted or non substituted benzimidazoles, one or several salts of benzimidazoles, any enantiomer of these benzimidazoles, one or several salts of enantiomer (s), any isomer of these benzimidazoles, any derivate of benzimidazole, any free base of benzimidazole or any mixing of these active principles.

The proton pump inhibitor used in the dosage forms of the invention may be used in neutral form or in the form of an alkaline salt, such as for instance the Mg++, Ca++, Na++, K++ or Li++ salts, preferably the Mg++ salts. Further where applicable, the compounds listed above may be used in racemic form or in the form of a substantially pure enantiomer thereof, or alkaline salts of the single enantiomers.

For example, the proton pump inhibitor concerned by the invention, are notably the proton pump inhibitor described pages 7 to 11 ofWO-A-97/25066, this extract being incorporated by reference in the present text.

The WO-A-2004/035020 patent application gives also a general formula of the class of benziimidazoles: pages 35-48. This extract of WO-A-2004/035020 is incorporated by reference in its entirety in the present text.

Examples of proton pump inhibitor are: esomeprazole, leminoprazole, omeprazole, pantoprazole, pariprazole, rabeprazole, timoprazole, picoprazole and tenatoprazole.

Suitable proton pump inhibitors are for example disclosed in EP-A1-0005129, EP-A1-174 726, EP-A1-166 287, GB 2 163 747 and WO90/06925, WO91/19711, WO91/19712, and further especially suitable compounds are described in WO95/01977 and WO094/27988.

The gastric acid suppressing agent is preferably a proton pump inhibitor, but H₂ receptor antagonists such as ranitidine, cimetidine or famotidine may be used in the pharmaceutical compositions with an alginate as proposed in WO 95/017080 or together with antacid agent(s). A wide variety of antacid agent(s) and/or alginates may be used in combination with a suitable proton pump inhibitor in the fixed unit dosage form according to the present invention. Such antacid agents include for example aluminium hydroxide, calcium carbonate, magnesium hydroxide, magnesium carbonate and aluminium magnesium hydroxide carbonate (hydrotalcit) taken alone or in combinations with each other. The alginates may be an alginate selected from alginic acid or sodium alginate or other pharmaceutically acceptable alginate salts, hydrates, esters etc. Especially preferred antacid agents are magnesium or calcium based antacid agents and aluminium hydroxide/magnesium carbonate complex. Suitable antacid agents are for instance described in U.S. Pat. No. 5,409,709.

The preferred proton pump inhibitor in the form of a racemat, an alkaline salt or one of its single enantiomers, optionally in combination with antacid agent(s), can be a immediate release form and/or a controlled release (controlled release) form.

For example, the gastric releasing agent can be made to individually enteric or non enteric coating layered individual units (small beads, granules, microcapsules or pellets).

For example, the gastric acid suppressing agent - preferably a proton pump inhibitor (proton pump inhibitor)- can be designed in the form of controlled release/MR microcapsules, notably of the type of the controlled release/MR acetylsalicylic acid microcapsules, as described herein.

It could be very useful that the oral pharmaceutical formulation according to the invention, comprises at least one active principle different from acetylsalicylic acid and gastric acid suppressing agent - preferably a proton pump inhibitor (proton pump inhibitor)-.

According to one embodiment, the active principle different from acetylsalicylic acid and gastric acid suppressing agent is selected in the group comprising: anti-platelet drugs, beta adrenergic receptor blockers, calcium channel blockers, angiotensin converting enzyme inhibitors, diuretics, anti-arrhythmic drugs, anti-ischemic drugs, anti-hypertensive drugs, beta adrenergic agonists, cardiac glycosides, nitrates, sodium channel blockers, central nervous system acting anti-hypertensive drugs, potassium channel activators, vasodilatory drugs, and vasoconstrictive drugs.

Examples of anti-platelet drugs include non-steroidal anti-inflammatory drugs, dipyridamole, and ticlopidine.

Examples of diuretics include acetazolamide, dichlorphenamide, methazolamide, glycerin, isosorbide, mannitol, urea, furosemide, bumetanide, ethacrynic acid, torsemide, azosemide, muzolimine, piretanide, tripamide, bendroflumethiazide (naturetin), benzthiazide (exna), chlorothiazide (diuril), hydrochlorothiazide (hydrodiuril), hydroflumethiazide (saluron), methyclothiazide (enduron), polythiazide (renese), trichlormethiazide, chlorthalidone (hygroton), indapamide (lozol), metolazone (mykrox, zaroxolyn), quinethazone (hydromox), amiloride, triamterene, spironolactone, canrenone, potassium canrenoate.

Examples of angiotensin converting enzyme inhibitors include benazepril, captopril, enalapril, fosinopril sodium, lisinopril, quinapril, ramipril, spirapril.

Examples of nitrates include amyl nitrite (isoamyl nitrite), nitroglycerin (glyceryl trinitrate, nitro-bid, nitrostat, nitrol, nitro-dur, others), isosorbide dinitrate (isordil, sorbitrate, dilatrate, others), isosorbide-5-mononitrate (imdur, ismo, others), erythrityl tetranitrate (cardilate).

Examples of calcium channel blockers include amlodipine (norvasc), bepridil (vascor), diltiazem (cardizem, dilacor), felodipine (plendil), isradipine (dynacirc), nicardipine (cardene), nifedipine (adalat, procardia), nimodipine (nimotop), verapamil (calan, isoptin, verelan).

Examples of vasodilator drugs include nitrovasodilators such as nitroglycerin, isosorbide dinitrate, sodium nitroprusside; angiotensin receptor antagonist such as losartan (cozaar), phosphodiesterase inhibitors such as amrinone (inocor), milrinone (primacor), and vesnarinone; "direct" vasodilators such as hydralazine (apresoline), nicorandil, adrenergic receptor antagonists such as prazosin (minipress, and other quinazoline derivatives), phentolamine (regitine), labetalol (normodyne, trandate), carvedilol, and bucindolol; Ca²⁺ channel blocking drugs such as nifedipine (adalat, procardia), amlodipine (norvasc), and sympathomimetics such as dobutamine (dobutrex).

Examples of anti-arrhythmic drugs include adenosine, amiodarone, bretylium, digoxin, digitoxin, diltiazem, disopyramide, esmolol, flecainide, lidocaine, mexiletine, moricizine, phenytoin, procainamide (N-acetyl procainamide), propafenone, propranolol, quinidine, sotalol, tocainide, verapamil.

According to another embodiment, the active principle different from acetylsalicylic acid and gastric acid suppressing agent is selected in the group of the anti-inflammatory drugs.

According to another embodiment, the active principle different from acetylsalicylic acid and gastric acid suppressing agent - preferably a proton pump inhibitor (proton pump inhibitor)-, is selected in the group of the Non Steroidal Anti Inflammatory Drugs (NSAIDs).

Regarding NSAIDs, very large number of patients who are treated with common NSAIDs or with more specific NSAIDs such as COX-2 inhibitors, have severe side effects, including life threatening ulcers and thrombotic cardiovascular events, that limit their therapeutic potential. In addition, there is evidence that patients with chronic inflammatory conditions, such as rheumatoid arthritis and systemic lupus erythematosis are at increased risk for thrombotic cardiovascular events. Thus, it is desirable that these patients receive antiplatelet therapy, with only minimal side effects. This need is reinforced by the fact that many patients treating with NSAIDs or suffering from chronic COX-2 mediated disease or condition, are elderly and thus are at increased risk for thrombotic cardiovascular events.

There is a need to associate anti-platelet aggregation drugs to NSAIDs, particularly COX-2 inhibitor anti-inflammatory drugs, without inducing gastric side effects.

Then, it has been proposed to associate low dose aspirin with COX-2 inhibitors. But due to the aspirin dilemma, the gastro-protection activity of the prostacyclin and of the prostaglandins, is affected and thus it induces severe gastric disorders.

The last mentioned embodiment according to the invention, offers an advantageous solution to this problem.

Preferably, the NSAID(s) is (are) selected in the group comprising:
- aminoarylcarboxylic acid and its derivates such as: enfenamic acid, flufenamic acid, isonixin, meclofenamic acid, mefenamic acid, morniflumate, niflumic acid & tolfenamic acid,
- arylacetic acid and its derivates such as: aceclofenac, acemetacin, amfenac, bromfenac, cimmetacin, diclofenac, etodolac, fentiazac, glucametacin, indomethacin, lonazolac, l'acid metiavinic, oxametacine, pirazolaque, proglumetacin, sulindac, tiaramide, tolmetin et zomepirac,
- arylcarboxylic acids such as ketorolac & tinoridine,
- arylpropionic acid and its derivates such as: alminoprofen, bermoprofen, carprofen, dexibuprofen, fenbufen, fenoprofen, flunoxaprofen, flurbiprofen, ibuprofen, ibuproxam, ketoprofen, loxoprofen, naproxen, oxaprozin, pranoprofen, protizinic acid & tiaprofenic acid,
- pyrazoles, *e.g*.: epirizole,
- pyrazolones such as: benzpiperylon, mofebutazone, oxyphenbutazone, phenylbutazone & ramifenazone,
- salicylic acid derivates such as : acetaminosalol, benorylate, eterisalate, fendosal, imidazole salicylate, lysine acetylsalicylate, morpholine salicylate, parsalmide, l'acid salamidacetic & le salsalate,
- thiazinecarboxamides such as : ampiroxicam, droxicam, lornoxicam, meloxicam, piroxicam & tenoxicam,
- others such as: bucillamine, bucolome, bumadizon, diferenpiramide, ditazol, emorfazone, nabumetone, nimesulide, proquazone et piroxicam (in the form of the betacyclodextrin complex).
- alclofenac, azapropazone, benoxaprofen, acid bucloxic, choline magnesium trisalicylate, clidanaque, clopinaque, dapsone, diflunisal, fenclofenec, floctafenine, flufenisal, (r)-flurbiprofen, (s)-flurbiprofen, furofenaque, feprazone, fluprofen, ibufenaque, indoprofen, isoxepac, isoxicam, miroprofen, mefenamic, meclofen, acid niflumic, nitroflurbiprofen, oxipinaque, podophyllotoxin derivates, piprofen, pirprofen, prapoprofen, sudoxicam, suprofen, acid tiaprofenic, tiopinaque, tioxaprofen, zidometacin, acid 2-fluoro-a-methyl[1,1'-biphenyl]-4-acetic, a 4-(nitrooxy)butyl ester, ketoporfen, ketrolac.

More preferably, the NSAID(s) is (are) selected in the group comprising: lornoxicam, diclofenac, nimesulide, ibuprofen, piroxicam, piroxicam (betacyclodextrin), naproxen, ketoprofen, tenoxicam, aceclofenac, indometacin, nabumetone, acemetacin, morniflumate, meloxicam, flurbiprofen, acid tiaprofenic, proglumetacin, acid mefenamic, fenbufen, etodolaque, tolfenamic acid, sulindac, phenylbutazone, fenoprofen, tolmetin, acetylsalicylic acid, dexibuprofen and/or the pharmaceutical salts and/or the complexes and/or the prodrugs and/or the mixtures thereof.

The examples of cyclooxygenase-2 selective inhibitors include: rofecoxib (VIOXX® cf. US-B- 5 474 995), etoricoxib (ARCOXIA™ cf. US-B- 5 861 419), celecoxib (CELEBREX® cf. US-B- 5 466 823), valdecoxib (cf. US ―B-6 633 272), parecoxib (cf. brevet US ―B-5 932 598), COX-189 (Novartis), BMS347070 (Bristol Myers Squibb), tiracoxib ou JTE522 (Japan Tobacco), ABT963 (Abott), CS502 (Sankyo), and GW406381 (GlaxoSmithKline).

According to another embodiment, the active principle different from acetylsalicylic acid and gastric acid suppressing agent - preferably a proton pump inhibitor (proton pump inhibitor)-, is selected in the group of the anti-diabetic drugs comprising: Acarbose, Acetohexamide, Buformin, 1-Butyl-3-metanilylurea, Carbutamide, Chlorpropamide, Ciglitazone, Glibornuride, Gliclazide, Glimepiride, Glipizide, Gliquidone, Glisoxepid, Glyburide, Glybuthiazole, Glybuzole, Glyhexamide, Glymidine, Glypinamide, Metformin, Miglitol, Nateglinide, Phenbutamide, Phenformin, Pioglitazone, Proinsulin, Repaglinide, Rosiglitazone, Tolazamide, Tolbutamde, Tolcyclamide, Troglitazone and/or the pharmaceutical salts and/or the complexes and/or the prodrugs and/or the mixtures thereof.

Advantageously, the controlled release-acetylsalicylic acid microcapsules are so designed that the absorption takes place over a period of between 24 and 48 hours in the following manner: 10% of the absorbed fraction of the dose at t=0.4 to 5 hours, 50% of the absorbed fraction of the dose at t=3.9 to 25 hours, and 90% of the absorbed fraction of the dose at t=23 to 45 hours.

In the disclosure of the invention, the term "controlled release-acetylsalicylic acid microcapsules" denotes microparticles of acetylsalicylic acid that are film-coated with at least one coating for modified/controlled release of acetylsalicylic acid. The non-film-coated microparticles of acetylsalicylic acid may, for example, be neutral cores coated with at least one layer containing acetylsalicylic acid, or microparticles of pure acetylsalicylic acid or alternatively granules formed by a matrix of support excipients including lansoprazole.

Advantageously, the film-coating (or coating) covering(s) has(have) sufficient mechanical strength to prevent it(them) tearing and/or it(them) breaking up in the organism, until the end of the release of the active principle. These controlled release-acetylsalicylic acid microcapsules can be compared to vehicles for the transport and the release of acetylsalicylic acid and, optionally, of one or more other active principles in the stomach and in the small intestine.

The pharmaceutical formulation according to the invention can also be characterized in that the controlled release-acetylsalicylic acid microcapsules have an in vitro release profile, in 0.05M potassium dihydrogenophosphate/sodium hydroxide buffer medium pH 6.8, such that:
- 70% of the acetylsalicylic acid is released over a period of time of between 1 and 10 hours, preferably between 2 and 8 hours, and even more preferably between 2 and 6 hours, and
- 40% of the acetylsalicylic acid is released over a period of time of between 0.5 and 5 hours, preferably between 1 and 4 hours, and even more preferably between 1 and 3 hours.

Advantageously, the controlled release-acetylsalicylic acid microcapsules have an in vitro release profile, in a 0.04M hydrochloric acid medium pH 1.4, such that 40% of the acetylsalicylic acid is released over a period of time of less than or equal to 3h, preferably less than or equal to 2h, and even more preferably less than or equal to 0.75h.

According to another pharmacokinetic defmition of the pharmaceutical formulation according to the invention, the controlled release-acetylsalicylic acid microcapsules have an in vitro release profile in 0.05M potassium dihydrogenophosphate/sodium hydroxide buffer medium pH 6.8, such that, for any value of time t of between 2h and t(70%), preferably for any value of time t of between 1h and t(70%), the % of dissolved (released) acetylsalicylic acid is greater than or equal to 35 t / t(70%).

For example, the coating of the controlled release-acetylsalicylic acid microcapsules represents 5 to 50% by weight, based on the total mass of said microcapsules.

Preferably, the coating of the controlled release-acetylsalicylic acid microcapsules comprises at least one layer, which controls the modified release of agent, the composition of said layer is as follows:
A) at least one film-forming (co)polymer (A) that is insoluble in the fluids of the gastrointestinal tract;
B) optionally, at least one water-insoluble hydrophilic film-forming (co)polymer (B) that is insoluble in the fluids of the gastrointestinal tract, carrying groups that are ionized in the fluids of the gastrointestinal tract,
C) at least one (co)polymer (C) that is soluble in the fluids of the gastrointestinal tract;
D) at least one plasticizer (D);
E) optionally, at least one surfactant and/or lubricant (E).

According to a preferred embodiment of the invention:
* (A) is selected from the group of following products:
   - non-water-soluble derivatives of cellulose, preferably ethylcellulose and/or cellulose acetate,
   - polyvinyl acetates,
   - and mixtures thereof.
* (B) is chosen from water-insoluble charged acrylic derivatives, preferably from (co)polymers of acrylic and methacrylic acid ester carrying at least one quaternary ammonium group, (B) even more preferably comprising at least one copolymer of alkyl (meth)acrylate and of trimethylammonioethyl methacrylate chloride, and more precisely the products sold under the trade marks EUDRAGIT ® RS and/or RL, e.g. the powders EUDRAGIT ® RL PO and/or EUDRAGIT ® RS PO and/or the granules EUDRAGIT ® RL 100 and/or EUDRAGIT ® RS 100 and/or the suspensions and/or solutions of these EUDRAGIT ® RL and RS, namely, respectively, EUDRAGIT® RL 30D and/or EUDRAGIT® RS 30D and/or EUDRAGIT® RL 12.5 and/or EUDRAGIT® RS 12.5;
* (C) is chosen from
   - nitrogenous (co)polymers, preferably from the group comprising polyacrylamides, poly-N-vinylamides, polyvinylpyrrolidones (PVP) and poly-N-vinyllactams;
   - water-soluble derivatives of cellulose,
   - polyvinyl alcohols (PVAs),
   - polyoxyethylenes (POEs),
   - and mixtures thereof;
      polyvinylpyrrolidone being particularly preferred.
* (D) is chosen from the group comprising:
   - cetyl alcohol esters,
   - glycerol and its esters, preferably from the following subgroup: acetylated glycerides, glyceryl monostearate, glyceryl triacetate, glyceryl tributyrate,
   - phthalates, preferably from the following subgroup: dibutyl phthalate, diethyl phthalate, dimethyl phthalate, dioctyl phthalate,
   - citrates, preferably from the following subgroup: acetyl tributyl citrate, acetyltriethyl citrate, tributyl citrate, triethyl citrate,
   - sebacates, preferably from the following subgroup: diethyl sebacate, dibutyl sebacate,
   - adipates,
   - azelates,
   - benzoates,
   - plant oils,
   - fumarates, preferably diethyl fumarate,
   - malates, preferably diethyl malate,
   - oxalates, preferably diethyl oxalate,
   - succinates, preferably dibutyl succinate,
   - butyrates,
   - salicylic acid,
   - triacetin,
   - malonates, preferably diethyl malonate,
   - castor oil (this being particularly preferred),
   - and mixtures thereof.
* (E) is chosen from the group comprising:
   - anionic surfactants, preferably from the subgroup of alkali metal or alkaline-earth metal salts of fatty acids, stearic acid and/or oleic acid being preferred,
   - and/or nonionic surfactants, preferably from the following subgroup:
      o polyoxyethylenated oils, preferably polyoxyethylenated hydrogenated castor oil,
      o polyoxyethylene-polyoxypropylene copolymers,
      o polyoxyethylenated esters of sorbitan,
      o polyoxyethylenated derivatives of castor oil,
      o stearates, preferably calcium stearate, magnesium stearate, aluminium stearate or zinc stearate,
      o stearyl fumarates, preferably sodium stearyl fumarate,
      o glyceryl behenates,
      o and mixtures thereof.

According to a particularly advantageous embodiment, the composition of the modified-release layer is as follows:
A. the film-forming polymer(s) (A) is (are) present in a proportion of 10 to 90%, preferably 20 to 40% by weight on a dry basis relative to the total mass of the coating composition;
B. the water-insoluble hydrophilic film-forming polymer(s) (B) is (are) present in a proportion of 10 to 90%, preferably 20 to 40% by weight on a dry basis relative to the total mass of the coating composition;
C. the polymer(s) (C) that is (are) soluble in the fluids of the gastrointestinal tract is (are) present in a proportion of 2 to 25, preferably 5 to 15% by weight on a dry basis relative to the total mass of the coating composition;
D. the plasticizer(s) (D) is (are) present in a proportion of 2 to 20, preferably of 4 to 15% by weight on a dry basis relative to the total mass of the coating composition;
E. the optional surfactant(s) and/or lubricant(s) (E) is (are) present in a proportion of 2 to 20, preferably of 4 to 15% by weight on a dry basis relative to the total mass of the coating composition.

For further details, in particular qualitative and quantitative details, regarding at least some of the constituents of this coating composition, reference will be made, for example, to European patent EP-B-0 709 087 or to PCT applications WO-A-2004/010983 and WO-A-2004/010984, the content of which is incorporated into the present disclosure in their entirety by way of reference.

The monolayer or multilayer coating may comprise various other additional adjuvants conventionally used in the coating field. They may be, for example, pigments or coloring agents, fillers, anti-foaming agents...

To prevent the problems of caking of the coated particles constituting the controlled release-acetylasalicylic acid microcapsules used in the invention, provision is advantageously made for adding thereto at least one anticaking agent preferably formed of talc, colloidal silica or a mixture of the two.

According to a particular embodiment of the invention, the controlled release-acetylasalicylic acid microcapsule coating responsible for the modified release of acetylasalicylic acid consists of a single coating layer or a single coating film. This simplifies their preparation and limits the degree of coating.

Moreover, the pharmaceutical formulation according to the invention has the particularity that the coating of each controlled release-acetylasalicylic acid microcapsule is nonenteric and does not desintegrate whatever the pH be, and in particular at any pH above 5.0.

Advantageously, the diameter of the controlled release-acetylasalicylic acid microcapsules is less than or equal to 1000 µm, preferably between 50 and 800 µm, and even more preferably between 100 and 600 µm. The microparticle diameters to which the present disclosure refers are, unless otherwise indicated, mean diameters by volume.

This size makes it possible to cross the stomach independently of the opening of the pylorus. The gastric transit time is thus shorter and more uniform.

Advantageously, the controlled release-acetylasalicylic acid microcapsules are obtained from particles of acetylasalicylic acid having a size of between 250 and 800 µm before the coating operation.

According to the invention, practical implementations in which the proportion of acetylasalicylic acid in the microcapsules (expressed as % by weight on a dry basis relative to the total mass of the microcapsules) is between 5 and 80, preferably between 10 and 60, and even more preferably between 20 and 50, are preferred.

As regards the monolayer or multilayer coating (or film) responsible for the modified release of acetylasalicylic acid, it represents, for example, at most 40%, preferably at most 15%, by weight of the microcapsules.

The controlled release-acetylasalicylic acid microcapsules are obtained from particles of acetylasalicylic acid which are coated by being sprayed with the intimate combination forming the coating, suspended in an organic solvent or mixture of organic solvents. The coating process, which constitutes a further subject of the invention, fits into the general pattern of microencapsulation techniques, of which the main ones are summarized in the article by C. DUVERNEY and J. P. BENOIT in "L'actualité chimique", December 1966. More precisely, the technique in question is microencapsulation by film coating. Preferably, this process consists essentially in: a) preparing the coating composition by mixing ABCDE in a solvent system, b) applying the composition/solvent system mixture to particles of acetylsalicylic acid, c) drying the resulting microcapsules, and d) if appropriate, mixing the latter with at least one anticaking agent. Examples of solvents which are suitable for forming part of the composition of the solvent system are ketones, esters, chlorinated solvents, alcohols, preferably aliphatic alcohols, alkanes or mixtures thereof. These solvents are advantageously C₁-C₆ compounds and particularly preferably acetone, methyl ethyl ketone, methanol, ethanol, isopropanol, cyclohexane and methylene chloride. If the coating methodology which can be used according to the invention is considered in greater detail, it can be stated that the coating composition/solvent system mixture is applied by being sprayed onto the moving particles of acetylasalicylic acid, said movement preferably being created by mechanical agitation or by blowing (fluidization). To obtain microcapsules according to the invention possessing the desired absorption kinetics, it is necessary to encapsulate particles of acetylasalicylic acid with a mean size of between 75 and 500 µm, preferably of between 300 and 500 µm, for a dose of between 75 and 320 mg.

The controlled release-acetylasalicylic acid microcapsules described above, which may have been obtained by the process also explained above, can be used for the preparation of novel galenical forms of aspirin having a biochemical selectivity for the inhibition of thromboxane relative to the other prostaglandins, in particular for the preparation of novel galenical forms useful as platelet aggregation inhibitors, and/or, more precisely, for the preparation of novel galenical forms active in the prevention and/or treatment of cardiovascular diseases and risks.

The present invention further relates to these novel galenical units as such, and comprising the pharmaceutical formulation according to the invention.

Advantageously, these galenical, novel in their structure, their presentation and their composition, are presented in the form of a sachet of powder, a sachet of a powder for multidose suspension to be reconstituted, a tablet or a gelatin capsule. They can contain, for instance, a dose of acetylasalicylic acid of 20 to 500 mg, preferably 50 to 400 mg and particularly preferably 50 to 325 mg of acetylasalicylic acid and a dose of proton pump inhibitor of 1 to 300 mg, preferably 2 to 200 mg and particularly preferably 5 to 120 mg. Such galenical forms are preferably administered per or in single or twice daily doses D,D'.

It should be noted that it can be of value to mix, in one and the same gelatin capsule, tablet or powder, at least two types of microcapsules whose absorption kinetics are different but within the framework characteristic of the controlled release-acetylasalicylic acid microcapsules according to US-B-5,603,957 (profile of curve of FIG. 1).

The invention will be understood more clearly from the following Examples, which are given solely by way of illustration and serve to provide a clear understanding of the invention and to illustrate its different embodiments and/or modes of implementation, as well as its various advantages.

### EXAMPLES

### Description of the figures:

Figure 1: In vitro release profiles for the controlled release-acetylasalicylic acid-based Microcapsules prepared according to Example 1.
Figure 2: In vitro release profiles for the controlled release-omeprazole based Microcapsules prepared according to Example 2.

### Example 1 Preparation of CR-ASA-based Microcapsules

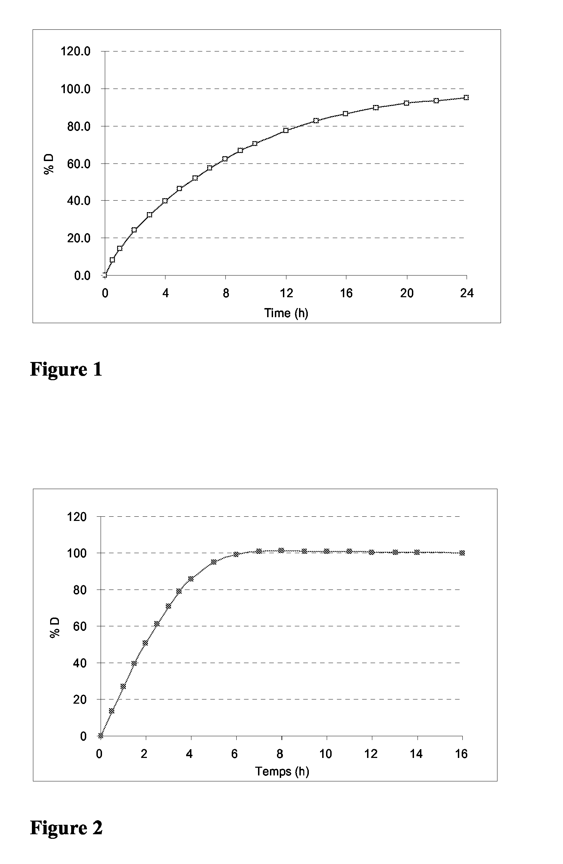

66 g of ethyl cellulose (Ethocel 7 Premium / Dow), 7 g of Plasdone K29/32® (Povidone/ISP), 8 g of castor oil, 9 g of magnesium stearate and 10 g tartaric acid are dispersed in 1200 g of a mixture made of 60% of isopropanol & 40% of acetone. The suspension is sprayed on 900 g of acetylsalicylic acid (aspirin) crystals, previously sieved between 200 and 500 µm.
These microparticles have been tested in a pH 6.8 (KH₂PO₄ 0.05M/ NaOH) dissolution medium maintained at 37°C and stirred with a paddle speed of 100 rpm (USP II apparatus). See Figure 1.

### Example 2 Preparation of CR- omeprazole- based Microcapsules

### step 1 :

700 g of omeprazole and 100 g de Klucel EF® (Hydroxypropyl cellulose / Aqualon) are dispersed in 3000 g of isopropanol. The suspension is sprayed on 200 g of neutral microspheres (Asahi-Kasei) in a spray coater Glatt GPCG1.

### step 2 :

50 g of ethyl cellulose (Ethocel 20 Premium / Dow), 20 g of Plasdone K29/32® (Povidone/ISP), 20 g of Lutrol F-68 (Poloxamer 188 / BASF) and 10 g of castor oil are dispersed in mixture made of 60% of isopropanol and 40% of acetone. This solution is sprayed on 900 g of omeprazole granules (prepared at step 1).
The obtained microparticles are filled into a gelatine size 3 capsule. The dose of omeprazole per capsule is, in this test, 80 mg i.e 127 mg of microcapsules.
These microcapsules have been tested in a pH 6.8 (KH₂PO₄ 0.05M/ NaOH) dissolution medium maintained at 37°C and stirred with a paddle speed of 100 rpm (USP II apparatus).
See Figure 2.

### Example 3: Preparation of IR- lansoprazole based Microcapsules

900 g of lansoprazole & 100 g of Klucel EF® (Hydroxypropyl cellulose / Aqualon) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.
These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Example 4: Preparation of enteric coated- lansoprazole based Microcapsules

### Step 1:

900 g of lansoprazole & 100 g of Klucel EF® (Hydroxypropyl cellulose / Aqualon) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.

### Step 2:

50 g of Eudragit L100-55® (Rohm) and 10 g of triethyl citrate are dispersed in isopropanol. This solution is sprayed on 450 g of lansoprazole granules (prepared at step 1).
These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Example 5: Preparation of IR- celecoxib based Microcapsules

860 g of celecoxib, 70 g of Klucel EF® (Hydroxypropyl cellulose / Aqualon) & 70 of Lutrol F-68 (Poloxamer 188 / BASF) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.
These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Example 6: Capsule containing CR-aspirin and CR-omeprazole

180 mg of microcapsules of acetylsalicylic acid prepared in example 1 (i.e.162.5 mg of acetylsalicylic acid) and 15.4 mg of microcapsules of omeprazole (i.e.10 mg of omeprazole) prepared at example 2 are filled in size 2 capsule.
This capsule is the final form of the drug for preventing cardiovascular diseases (by means of aspirin), while hindering gastric damages due to the presence of a ppi and CR-ASA microcapsules.

### Example 7: Capsule containing CR-aspirin and IR-lansoprazole

180 mg of microcapsules of acetylsalicylic acid prepared in example 1 (i.e.162.5 mg of acetylsalicylic acid) and 5.5 mg of microcapsules of lansoprazole (i.e.5 mg of lansoprazole) prepared at example 3 are filled in size 2 capsule.
This capsule is the final form of the drug for preventing cardiovascular diseases (by means of aspirin), while hindering gastric damages due to the presence of a ppi and CR-ASA microcapsules.

### Example 8: Capsule containing CR-aspirin and enteric coated-lansoprazole

180 mg of microcapsules of acetylsalicylic acid prepared in example 1 (i.e.162.5 mg of acetylsalicylic acid) and 6.2 mg of microcapsules of lansoprazole (i.e.5 mg of lansoprazole) prepared at example 4 are filled in size 2 capsule.
This capsule is the final form of the drug for preventing cardiovascular diseases (by means of aspirin), while hindering gastric damages due to the presence of a ppi and CR-ASA microcapsules.

### Example 9: Capsule containing CR-aspirin and enteric coated-lansoprazole and celecoxib

180 mg of microcapsules of acetylsalicylic acid prepared in example 1 (i.e.162.5 mg of acetylsalicylic acid), 6.2 mg of microcapsules of lansoprazole (i.e.5 mg of lansoprazole) prepared at example 4 and 233 mg of microcapsules of celecoxib (i.e.200 mg of celecoxib) prepared at example 5 are filled in size 0 capsule.
This capsule is the final dosage form for preventing cardiovascular diseases (by means of aspirin), said diseases being caused by repeated administration of anti-inflammatory drugs, such as COX-2 inhibitors (celecoxib), while hindering gastric damages due to the presence of a ppi and CR-ASA microcapsules. The combination of a ppi with the CR-ASA microcapsules makes it possible to prevent gastric damages due to aspirin.

### Comparative example 10 showing that the known CR-ASA microcapsules could be improved, by means of the combination according to the invention:

Controlled release microcapsules of aspirin according to example 1 were compared to aspirin at a dose of 325 mg in double blind, randomized cross-over study, on 24 healthy non smoking volunteers. Endoscopic damage was assessed on days 0, 7, 14, 21 on each treatment period. The primary end point was the total number of gastrioduodenal erosions and petechiae assessed endoscopically. The study performed by a group led by Professor Hawkey of Gastroenterology Division of University Hospital, Nottingham (UK) indicated that Controlled released aspirin cause less endoscopic damage than conventional aspirin.

The study indicated that significantly fewer gastric lesions were observed in patients taking controlled released aspirin 325 mg than in patients taking entero-coated aspirin at the same dose. In particular, gastric erosion per patient were 1.57 with Controlled released aspirin 325 compared to 5.48 with entero-coated aspirin product, or a reduction of 70% (p<0.001). Concerning haemorrhagic event, 0.3 events per patient were observed with controlled released aspirin 325 compared to 2.96 with conventional aspirin (p<0.001). In addition, a 3.09 petechia per patient were observed with controlled released aspirin compared to 7.35 with the comparator (p<0.001).

These very positive results for controlled released aspirin could be explained by the biochemical selectivity of the product which inhibits platelets COX-1 and consequently thromboxan (TXB2), the platelet aggregant prostanoids, which sparing prostacyclin (PGI2), the systemic cytoproctective prostaglandin generated by endothelial COX-1.

Although, this result is very positive for GI tract safety, it open the door to an improvement of the controlled released aspirin directed toward a decrease of the level of safety events especially those observed in gastro intestinal tract. It is worthwhile noticing that if the number of adverse events is significantly decreased using Controlled released aspirin compared to conventional aspirin, the GI tact events (i.e. erosion and petechia) are still measurable and could led to some safety concerns for chronic use.

The embodiments illustrated and discussed in this specification are intended only to teach those skilled in the art the best way known to the inventors to make and use the invention. Nothing in this specification should be considered as limiting the scope of the present invention. Modifications and variations of the above-described embodiments of the invention are possible without departing from the invention, as appreciated by those skilled in the art in light of the above teachings. It is therefore to be understood that, within the scope of the claims and their equivalents, the invention may be practiced otherwise than as specifically described, which merely illustrate preferred embodiments.

## Claims

1. An oral pharmaceutical formulation for enhancing notably the safety of antithrombotic treatments,
■ comprising a combination of microcapsules for the controlled release of acetylsalicylic acid (ASA), in the gastrointestinal environment and at least one gastric acid suppressing agent,
■ said microcapsules:
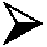 having an in vitro release profile, in 0.05M potassium dihydrogenophosphate/sodium hydroxide buffer medium pH 6.8, such that 70% of the ASA is released over a period of time of between 2 and 20 hours, preferably between 4 and 18 hours, and even more preferably between 6 and 15 hours, and
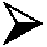 inhibiting COX-1 in portal vein which limits the production of thromboxane while maintaining COX-1 in systemic blood stream and hence limiting the inhibition of the production of prostaglandin and prostacyclin, to protect the gastric endothelium and to maintain its vasodilatation properties;
■ said gastric acid suppressing agent increasing the pH of the stomach just enough to minimize the damage resulting from the residual amount of non-deacetylated ASA coming from the liver and the portal blood circulation and entering the systemic blood circulation.

2. An oral pharmaceutical formulation according to claim 1, wherein the microcapsules being orally ingestible in a dose D and comprising particles of acetylsalicylic acid with a mean diameter below or equal to 1000 µm, preferably between 50 and 800 µm, and even more preferably between 100 and 600 µm which are coated and designed so that, when ingested orally in a single administration of a dose D of between 50 and 325 mg of ASA, they induce moderate ASA absorption kinetics in vivo in man, extending over at least 24 hours, said ASA absorption being less than or equal to 10% by weight of the absorbed fraction of D at a time t after ingestion of 0.4 hour, less than or equal to 50% by weight of the absorbed fraction of D at t=3.9 hours, and less than or equal to 90% by weight of the absorbed fraction of D at t=23 hours, t being given to within +/-10%.

3. An oral pharmaceutical formulation according to claim 1 or 2, wherein the gastric acid suppressing agent is a proton pump inhibitor (ppi).

4. An oral pharmaceutical formulation according to any of the preceding claims, wherein the dose D of ASA in the microcapsules is comprised between 60 and 320 mg, preferably between 75 and 310 mg.

5. An oral pharmaceutical formulation according to any of the preceding claims, wherein the dose D' of gastric acid suppressing agent is comprised between 1 and 130 mg, preferably between 2 and 120 mg.

6. An oral pharmaceutical formulation according to any of the preceding claims:
a) the dose D of ASA in the CR-ASA microcapsules is comprised between 50 and 325 mg;
b) the dose D' of gastric acid suppressing agent is comprised between 5 and 120 mg;
c) it is a once-a-day administration form.

7. An oral pharmaceutical formulation according to claim 6 including at least one immediate release gastric acid suppressing agent form.

8. An oral pharmaceutical formulation according to claim 6 including at least one controlled release gastric acid suppressing agent form.

9. An oral pharmaceutical formulation according to any of claims 1 to 5:
i. wherein the dose D of ASA in the microcapsules is comprised between 50 and 325 mg;
ii. wherein the dose D' of gastric acid suppressing agent is comprised between 5 and 120 mg;
iii. which is a twice-a-day administration form.

10. An oral pharmaceutical formulation according to claim 9 including at least one immediate release ASA form.

11. An oral pharmaceutical formulation according to claim 9 including at least one immediate release gastric acid suppressing agent form.

12. An oral pharmaceutical formulation according to claim 9 including at least one controlled release gastric acid suppressing agent form.

13. An oral pharmaceutical formulation according to any of the preceding claims, which is designed so that it induces reduction of bleeding and pitichia, notably, in the stomach during the treatment.

14. An oral pharmaceutical formulation according to any of the preceding claims, which is designed so that it improves the healing process, notably, in the stomach during the treatment.

15. An oral pharmaceutical formulation according to any of the preceding claims, comprising at least one active principle different from ASA and gastric acid suppressing agent.

16. An oral pharmaceutical formulation according to claim 15, wherein the active principle different from ASA and gastric acid suppressing agent - preferably a proton pump inhibitor (ppi)-, is selected in the group comprising:
■ the cardiovascular drugs acting as anti-platelets;
■ the cardiovascular drugs acting as beta-blockers;
■ the cardiovascular drugs acting as ACE inhibitors;
■ and any of their associations.

17. An oral pharmaceutical formulation according to claim 15, wherein the active principle different from ASA and gastric acid suppressing agent is selected in the group of the anti-inflammatory drugs.

18. An oral pharmaceutical formulation according to claim 17, wherein the active principle different from ASA and gastric acid suppressing agent is selected in the group of the Non Steroidal Anti Inflammatory Drugs (NSAIDs).

19. An oral pharmaceutical formulation according to claim 15, wherein the active principle different from ASA and gastric acid suppressing agent, is selected in the group of the anti-diabetic drugs.

20. An oral pharmaceutical formulation according to claim 2, wherein the ASA microcapsules are so designed that the absorption takes place over a period of between 24 and 48 hours in the following manner:
10% of the absorbed fraction of D at t=0.4 to 5 hours, 50% of the absorbed fraction of D at t=3.9 to 25 hours, and 90% of the absorbed fraction of D at t=23 to 45 hours.

21. An oral pharmaceutical formulation according to any of the preceding claims, wherein the coating agent of the ASA microcapsules represents 5 to 50% by weight, based on the total mass of said microcapsules.

22. An oral pharmaceutical formulation according to any of the preceding claims, wherein the coating of the ASA microcapsules comprises at least one layer which controls the modified release of agent, the composition of said layer is as follows:
A) at least one film-forming (co)polymer (A) that is insoluble in the fluids of the gastrointestinal tract;
B) optionally, at least one water-insoluble hydrophilic film-forming (co)polymer (B) that is insoluble in the fluids of the gastrointestinal tract, carrying groups that are ionized in the fluids of the gastrointestinal tract,
C) at least one (co)polymer (C) that is soluble in the fluids of the gastrointestinal tract;
D) at least one plasticizer (D);
E) optionally, at least one surfactant and/or lubricant (E).

23. An oral pharmaceutical formulation according to claim 22, wherein:
* (A) is selected from the group of following products:
• non-water-soluble derivatives of cellulose,
• polyvinyl acetates,
• mixtures thereof;
* (B), when it is present, is chosen from water-insoluble charged acrylic derivatives;
* (C) is chosen from
• nitrogenous (co)polymers;
• water-soluble derivatives of cellulose,
• polyvinyl alcohols (PVAs),
• polyoxyethylenes (POEs),
• and mixtures thereof;
* (D) is chosen from the group comprising:
• cetyl alcohol esters,
• glycerol and its esters,
• phthalates, citrates, sebacates, adipates,
• azelates,
• benzoates,
• plant oils,
• fumarates, malates, oxalates, succinates,
• butyrates,
• salicylic acid,
• triacetin,
• malonates,
• castor oil ,
• and mixtures thereof;
* (E) is chosen from the group comprising:
• anionic surfactants,
• and/or nonionic surfactants.

24. An oral pharmaceutical formulation according to claim 22, wherein the composition of the modified-release layer is as follows:
A. the film-forming polymer(s) (A) is (are) present in a proportion of 10 to 90%, by weight on a dry basis relative to the total mass of the coating composition;
B. the hydrophilic water-insoluble film-forming polymer(s) (B) is (are) present in a proportion of 0 to 90%, by weight on a dry basis relative to the total mass of the coating composition;
C. the soluble polymer(s) (C) is (are) present in a proportion of 2 to 25, by weight on a dry basis relative to the total mass of the coating composition;
D. at least one plasticizer (D) is (are) present in a proportion of 2 to 20, by weight on a dry basis relative to the total mass of the coating composition;
E. the optional surfactant(s) and/or lubricant(s) (E) is (are) present in a proportion of 2 to 20, by weight on a dry basis relative to the total mass of the coating composition.

25. An oral pharmaceutical formulation according to any of the preceding claims, wherein the diameter of the ASA microcapsules is less than or equal to 1000 µm.

26. An oral pharmaceutical formulation according to any of the preceding claims, wherein the ASA microcapsules are obtained from particles of ASA having a size of between 250 and 800 µm before the coating operation.

27. An oral pharmaceutical formulation according to any of the preceding claims, wherein the proportion of ASA in the microcapsules (expressed as % by weight on a dry basis relative to the total mass of the microcapsules) is between 5 and 80.

28. An oral pharmaceutical formulation according to any of the preceding claims, provided in the form a galenical unit selected in the group comprising: a sachet of powder, a sachet of a powder for multidose suspension to be reconstitued, a tablet or a gelatin capsule.

29. An oral pharmaceutical formulation comprising:
• at least one gastric acid suppressing agent, and
• acetylsalicylic acid that is coated to form microcapsules, said microcapsules having a release profile such that 70% of the acetylsalicylic acid in 0.05M potassium dihydrogenophosphate/sodium hydroxide buffer medium at a pH of 6.8 is released between about 2 and 20 hours, preferably between about 4 and 18 hours and even more preferably between 6 and 15 hours.
